# EUROPEAN PATENT SPECIFICATION

(11) **EP 4 031 221 B1**
(45) Date of publication and mention of the grant of the patent: **26.08.2026**
(21) Application number: 20882316.1
(22) Date of filing: 26.10.2020
(51) Int. Cl.: A61M 25/00, A61M 25/01, A61M 25/06, A61M 29/00, A61M 5/00

(54) **RAPIDLY INSERTABLE CENTRAL CATHETER AND METHODS THEREOF**
SCHNELL EINFÜHRBARER ZENTRALER KATHETER UND VERFAHREN DAFÜR
CATHÉTER CENTRAL À INSERTION RAPIDE ET MÉTHODES ASSOCIÉES

(30) Priority: 27.10.2019 US 201962926559 P
(43) Date of publication of application: 27.07.2022
(73) Proprietor: Bard Access Systems, Inc., Salt Lake City, UT 84116 (US)
(72) Inventor: HOWELL, Glade Harold, Draper, UT 84020 (US)
(74) Representative: Hoffmann Eitle
(86) International application number: PCT/US2020/057397
(87) International publication number: WO 2021/086793

(56) References cited:
- EP-A1- 2 896 424
- US-A- 5 370 615
- US-A- 5 718 678
- US-A1- 2005 283 221
- US-A1- 2008 045 894
- US-A1- 2010 256 487
- US-A1- 2015 320 969
- US-A1- 2015 359 549
- US-A1- 2019 255 294

## Description

### PRIORITY

This application claims the benefit of priority to U.S. Provisional Application No. 62/926,559, filed October 27, 2019.

### BACKGROUND

A central venous catheter ("CVC") is formed of a material having a relatively low durometer, which contributes to the CVC having a lack of column strength. Due to the lack of column strength, CVCs are commonly introduced into patients and advanced through vasculatures thereof by way of the Seldinger technique. The Seldinger technique utilizes a number of steps and medical devices (e.g., a needle, a scalpel, a guidewire, an introducer sheath, a dilator, a CVC, etc.). While the Seldinger technique is effective, the number of steps are time consuming, handling the number of medical devices is awkward, and both of the foregoing can lead to patient trauma. In addition, there is a relatively high potential for touch contamination due to the number of medical devices that need to be interchanged during the number of steps of the Seldinger technique. As such, there is a need to reduce the number of steps and medical devices involved in introducing a catheter into a patient and advancing the catheter through a vasculature thereof.
US 2005/0283221 A1 discloses a balloon catheter configured for rapid exchange and used to open up blockages. A rapid exchange catheter is one that is exchanged over two or more guidewires, a procedure of many steps. A section of the catheter is for a guidewire receiving lumen with an exit port.

Disclosed herein are rapidly insertable central catheters ("RICCs") and methods thereof that address the foregoing.

### SUMMARY

In order to achieve the above objects and effects, the technical solution implemented by the present invention is defined in the independent claims.
Disclosed herein is a RICC including, in some embodiments, a catheter tube, a suture wing disposed over a medial portion of the catheter tube, a hub coupled to a proximal-end portion of the catheter tube, and a number of extension legs extending from the hub equal to a number of lumens extending through the RICC. The catheter tube includes a first section in a distal portion of the catheter tube and a second section proximal of the first section. The second section of the catheter tube includes an outer layer of the catheter tube extruded over an inner layer of the catheter tube such that an outer diameter of the catheter tube is larger in the second section than the first section of the catheter tube.

In some embodiments, the inner layer of the catheter tube is formed of a first polymeric material having a first durometer and the outer layer of the catheter tube is formed of a second polymeric material having a second durometer less than the first durometer.

In some embodiments, the inner layer of the catheter tube is formed of a first polymeric material having a first durometer and the outer layer of the catheter tube is formed of a second polymeric material having a second durometer substantially equal to the first durometer.

In some embodiments, each polymeric material of the first and second polymeric materials is a polyurethane.

In some embodiments, each layer of the inner and outer layers of the catheter tube is formed of a same polymeric material.

In some embodiments, the polymeric material is a polyurethane.

In some embodiments, the catheter tube further includes a bump demarcating a third section of the catheter tube proximal of the second section. The third section has a larger outer diameter than both the first and second sections of the catheter tube.

In some embodiments, the suture wing is disposed over the bump of the catheter tube such that the catheter tube proximal of the suture wing has a larger outer diameter than the catheter tube distal of the suture wing.

In some embodiments, the catheter tube between the suture wing and the hub has a reverse taper in which the outer diameter of the catheter tube continuously increases from the suture wing to the hub.

In some embodiments, the RICC is a triluminal catheter including a trifurcated hub as the hub. The RICC also has three extension legs extending from the hub. Each extension leg of the three extension legs includes a Luer connector coupled to a proximal-end portion of the extension leg.

In some embodiments, the RICC includes a first lumen extending from an opening in a proximal end of a first Luer connector to an opening in a distal end of the first section of the catheter tube, a second lumen extending from an opening in a proximal end of a second Luer connector to a first eyelet in a distal portion of the second section of the catheter tube, and a third lumen extending from an opening in a proximal end of a third Luer connector to a second eyelet in the distal portion of the second section of the catheter tube.

In some embodiments, the RICC is a diluminal catheter including a bifurcated hub as the hub. The RICC also has two extension legs extending from the hub. Each extension leg of the two extension legs includes a Luer connector coupled to a proximal-end portion of the extension leg.

In some embodiments, the RICC includes a first lumen extending from an opening in a proximal end of a first Luer connector to an opening in a distal end of the first section of the catheter tube and a second lumen extending from an opening in a proximal end of a second Luer connector to an eyelet in a distal portion of the second section of the catheter tube.

In some embodiments, the catheter tube has a column strength sufficient to prevent buckling of the catheter tube when inserted into an insertion site and advanced through a vasculature of a patient.

Also disclosed herein is a catheter tube including, in some embodiments, a first section in a distal portion of the catheter tube and a second section proximal of the first section. The second section of the catheter tube includes an outer layer of the catheter tube extruded over an inner layer of the catheter tube such that an outer diameter of the catheter tube is larger in the second section than the first section of the catheter tube. The catheter tube has a column strength sufficient to prevent buckling of the catheter tube when inserted into an insertion site and advanced through a vasculature of a patient without use of the Seldinger technique.

In some embodiments, the inner layer of the catheter tube is formed of a first polymeric material having a first durometer and the outer layer of the catheter tube is formed of a second polymeric material having a second durometer less than the first durometer.

In some embodiments, the inner layer of the catheter tube is formed of a first polymeric material having a first durometer and the outer layer of the catheter tube is formed of a second polymeric material having a second durometer substantially equal to the first durometer.

In some embodiments, each polymeric material of the first and second polymeric materials is a polyurethane.

In some embodiments, each layer of the inner and outer layers of the catheter tube is formed of a same polymeric material.

In some embodiments, the polymeric material is a polyurethane.

In some embodiments, the catheter tube further includes a bump demarcating a third section of the catheter tube proximal of the second section. The third section has a larger outer diameter than both the first and second sections of the catheter tube.

In some embodiments, the catheter tube between the bump and a proximal end of the third section of catheter tube has a reverse taper in which the outer diameter of the catheter tube continuously increases from the bump to the proximal end of the third section of catheter tube.

In some embodiments, the catheter tube is a triluminal catheter tube including a first lumen extending from a first opening in a proximal end of the catheter tube to an opening in a distal end of the first section of the catheter tube, a second lumen extending from a second opening in the proximal end of the catheter tube to a first eyelet in a distal portion of the second section of the catheter tube, and a third lumen extending from a third opening in the proximal end of the catheter tube to a second eyelet in the distal portion of the second section of the catheter tube.

In some embodiments, the catheter tube is a diluminal catheter tube including a first lumen extending from a first opening in a proximal end of the catheter tube to an opening in a distal end of the first section of the catheter tube and a second lumen extending from a second opening in the proximal end of the catheter tube to an eyelet in a distal portion of the second section of the catheter tube.

Also disclosed herein is a method for manufacturing layered catheter tubes such as the foregoing catheter tube including, in some embodiments, an inner-layer forming step of forming the inner layer of the catheter tube by extruding monoluminal tubing of a first polymeric material. The method further includes an inserting step of inserting an end of the monoluminal tubing through a die of an extruder. The method further includes a second-layer forming step of forming the outer layer of the catheter tube by periodically forcing a melt of a second polymeric material through the die around the monoluminal tubing, thereby forming mixed-layer tubing having sections of the monoluminal tubing interspersed with sections of layered tubing. The method further includes a pulling step of pulling the mixed-layer tubing through a cooling bath with a puller to cool the mixed-layer tubing. The method further includes a cutting step of cutting the mixed-layer tubing in at least the sections of the monoluminal tubing with a cutter to form the layered catheter tubes.

In some embodiments, the method further includes a lumen forming step of forming one or more additional lumens to that of the monoluminal tubing by injecting air into the melt of the second polymeric material while forcing the melt of the second polymeric material through the die around the monoluminal tubing.

In some embodiments, the method further includes an eyelet-creating step of creating one or more eyelets in the sections of the layered tubing to correspondingly establish one or openings to the one or more additional lumens.

In some embodiments, the method further includes a bump-forming step of forming bumps in outer diameter in the sections of the layered tubing by periodically slowing a rate of pulling the mixed-layer tubing with the puller to increase the outer diameter after the bumps.

In some embodiments, the method further includes a reverse-tapering step of reverse tapering the outer diameter in the sections of the layered tubing after the bumps by continuously slowing the rate of pulling the mixed-layer tubing with the puller.

In some embodiments, the method further includes a bonding layer-applying step of applying a bonding layer over the monoluminal tubing before forcing the melt of the second polymeric material through the die around the monoluminal tubing.

In some embodiments, the first polymeric material has a first durometer and the second polymeric material has a second durometer less than the first durometer.

In some embodiments, the first polymeric material has a first durometer and the second polymeric material has a second durometer substantially equal to the first durometer.

In some embodiments, each polymeric material of the first and second polymeric materials is a polyurethane.

These and other features of the concepts provided herein will become more apparent to those of skill in the art in view of the accompanying drawings and following description, which describe particular embodiments of such concepts in greater detail.

### DRAWINGS

FIG. 1 illustrates an isometric view of a RICC in accordance with some embodiments.
FIG. 2 illustrates an exploded view of the RICC in accordance with some embodiments.
FIG. 3 illustrates a distal portion of a catheter tube of the RICC in accordance with some embodiments.
FIG. 4A illustrates a first transverse cross section of the catheter tube in accordance with some embodiments.
FIG. 4B illustrates a second transverse cross section of the catheter tube in accordance with some embodiments.
FIG. 4C illustrates a third transverse cross section of the catheter tube in accordance with some embodiments.
FIG. 4D illustrates a fourth or fifth transverse cross section of the catheter tube in accordance with some embodiments.
FIG. 5 illustrates a portion of a method of manufacturing the catheter tube in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

As set forth above, there is a need to reduce the number of steps and medical devices involved in introducing a catheter into a patient and advancing the catheter through a vasculature thereof. Disclosed herein are RICCs and methods thereof that address the foregoing.

### Rapidly insertable central catheters

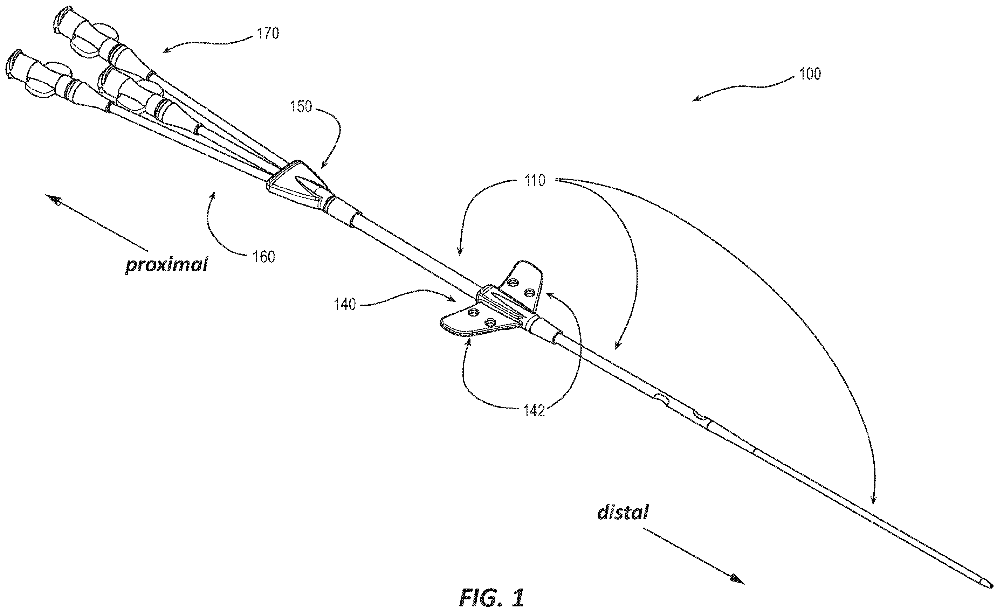

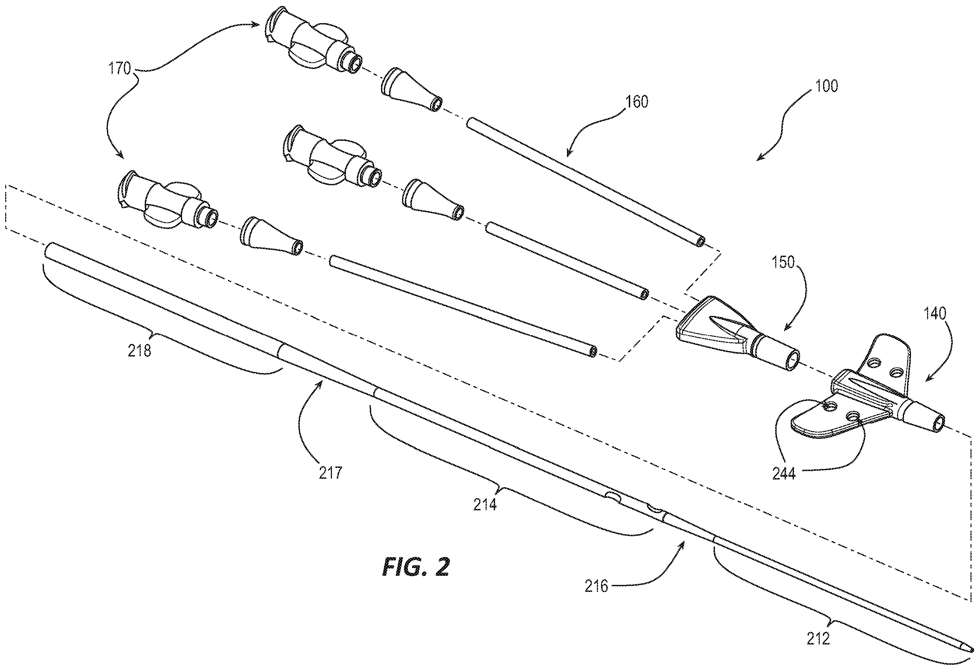

FIG. 1 illustrates an isometric view of a RICC 100 in accordance with some embodiments. FIG. 2 illustrates an exploded view of the RICC 100 in accordance with some embodiments.

As shown, the RICC 100 includes a catheter tube 110, a suture wing 140, a hub 150, and a number of extension legs 160 extending from the hub 150.

The suture wing 140 is disposed over a medial portion of the catheter tube 110 between a proximal portion and a distal portion of the catheter tube 110. The suture wing 140 includes a through hole longitudinally extending through the suture wing 140 configured for insertion of the catheter tube 110 therethrough. (*See,* for example, FIG. 2.)

The suture wing 140 includes a pair of wings 142 including a number of through holes 244 for suturing the suture wing 140 to a patient. Each wing of the pair of wings 142 can include one through hole, two through holes, three through holes, or four through holes for suturing the suture wing 140 to a patient.

The hub 150 is coupled to the proximal-end portion of the catheter tube 110 such as by insertion of the proximal-end portion of the catheter tube 110 into a bore in a distal-end portion of the hub 150. While not shown, the hub 150 also includes a number of bores in a proximal-end portion of the hub 150 corresponding in number to the number of extension legs 160. The number of bores in the distal-end portion of the hub 150 are configured to accept insertion of the number of extension legs 160 into the number of bores.

The RICC 100 can be a monoluminal catheter or a multiluminal catheter such as a diluminal catheter, a triluminal catheter, a tetraluminal catheter, a pentaluminal catheter, or a hexaluminal catheter. Accordingly, the hub 150 is either not furcated for the monoluminal catheter or furcated in accordance with a number of lumens extending through the RICC 100. For example, the hub 150 can be bifurcated for the diluminal catheter or trifurcated for the triluminal catheter. Depending upon a chosen method of manufacturing, the hub 150 can be molded over a number of core pins for a number of fluid pathways longitudinally extending through the hub 150 configured to fluidly connect the number of catheter-tube lumens of the catheter tube 110 to a number of extension-leg lumens of the number of extension legs 160. Alternatively, the hub 150 can be molded over a number of cannulas longitudinally extending through the hub 150 configured to fluidly connect the number of catheter-tube lumens of the catheter tube 110 to the number of extension-leg lumens of the number of extension legs 160.

The number of extension legs 160 extend from the hub 150 by way of their distal-end portions. The number of extension legs 160 is equal to the number of lumens extending through the RICC 100. For example: If the RICC 100 is a monoluminal catheter, one extension leg extends from the hub 150. If the RICC 100 is a diluminal catheter, two extension legs extend from the hub 150. If the RICC 100 is a triluminal catheter, three extension legs extend from the hub 150.

The RICC 100 further includes a number of Luer connectors 170 for fluidly connecting a number of medical devices to the RICC 100. Each extension leg of the number of extension legs 160 includes a Luer connector of the number of Luer connectors 170 coupled to a proximal-end portion of the extension leg. Given the foregoing, the number of Luer connectors 170 is equal to the number of extension legs 160, which number of extension legs 160, in turn, is equal to the number of lumens extending through the RICC 100. For example: If the RICC 100 is a monoluminal catheter, one extension leg extends from the hub 150 and one Luer connector is coupled to the one extension leg. If the RICC 100 is a diluminal catheter, two extension legs extend from the hub 150 and two Luer connectors are respectively coupled to the two extension legs. If the RICC 100 is a triluminal catheter, three extension legs extend from the hub 150 and three Luer connectors are respectively coupled to the three extension legs.

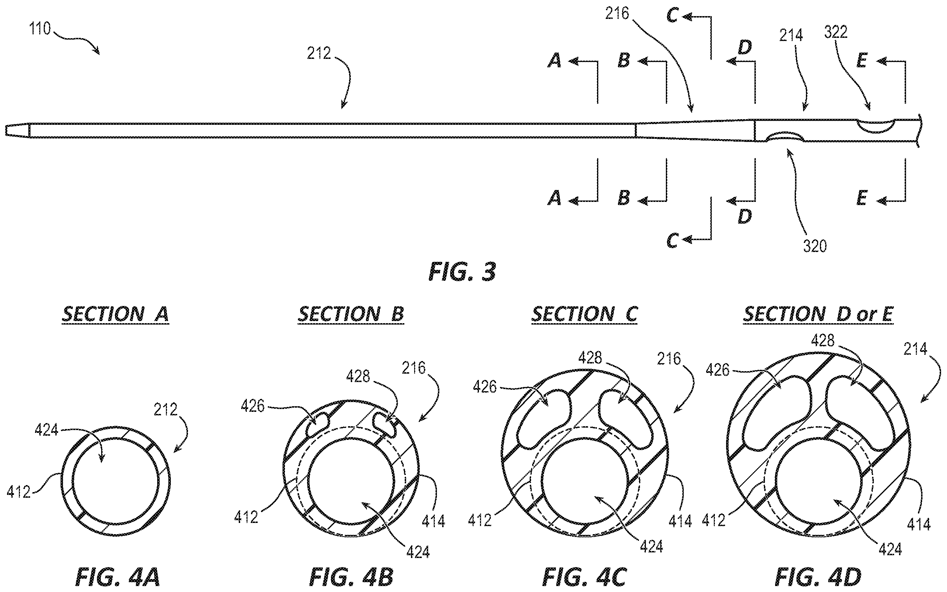

The catheter tube 110 includes at least a first section 212 in the distal portion of the catheter tube 110 and a second section 214 proximal of the first section 212 of the catheter tube 110. The catheter tube 110 can include a transition 216 between the first section 212 and the second section 214 of the catheter tube 110 in accordance with the method of manufacturing the catheter tube 110 set forth below. Indeed, in accordance with the manufacturing method set forth below, the second section 214 of the catheter tube 110 includes an outer layer 414 (*see* FIGS. 4A-4D) of the catheter tube 110 extruded over an inner layer 412 (*see* FIGS. 4A-4D) of the catheter tube 110 such that an outer diameter of the catheter tube 110 is larger in the second section 214 than the first section 212 of the catheter tube 110 commencing with the transition 216 between the first section 212 and the second section 214 of the catheter tube 110.

The inner layer 412 of the catheter tube 110 is formed of a first polymeric material (e.g., polytetrafluoroethylene, polypropylene, or a polyurethane) having a first durometer, while the outer layer 414 of the catheter tube 110 is formed of a second polymeric material (e.g., polyvinyl chloride, polyethylene, a polyurethane, or silicone) having a second durometer less than the first durometer, more than the first durometer, or substantially equal to the first durometer. For example, each layer of the inner layer 412 and the outer layer 414 of the catheter tube 110 can be made from a different polyurethane (e.g., a same or different diisocyanate or triisocyanate reacted with a different diol or triol, a different diisocyanate or triisocyanate reacted with a same or different diol or triol, etc.) having a different durometer. Indeed, polyurethane is advantageous for the catheter tube 110 in that polyurethane can be relatively rigid at room-temperature but become more flexible *in vivo* at body temperature, which reduces irritation to vessel walls and phlebitis. Polyurethane is also advantageous in that can be less thrombogenic than some other polymers.

It should be understood the first durometer and the second durometer can be on different scales (e.g., Type A or Type D). Thus, if the second durometer of the second polymeric material is less than the first durometer of the first polymeric material, the second durometer might not be numerically less than the first durometer. Likewise, if the second durometer of the second polymeric material is more than the first durometer of the first polymeric material, the second durometer might not be numerically more than the first durometer. That said, the hardness of the second polymeric material can still be less or more than the hardness of the first polymeric material as the different scales - each of which ranges from 0 to 100 - are designed for characterizing different materials in groups of the materials having a like hardness.

Notwithstanding the foregoing, the inner layer 412 and the outer layer 414 of the catheter tube 110 can be formed of a same polymeric material (e.g., a polyurethane) having substantially equal durometers provided a column strength of the catheter tube 110 is sufficient to prevent buckling of the catheter tube 110 when inserted into an insertion site and advanced through a vasculature of a patient.

The catheter tube 110 having at least the first section 212 of the first polymeric material and the second section 214 having the inner layer 412 of the first polymeric material and the outer layer 414 of the second polymeric material has a column strength sufficient to prevent buckling of the catheter tube 110 when the catheter tube 110 is inserted into an insertion site and advanced through a vasculature of a patient. The column strength of the catheter tube 110 is notable in that it makes it possible to rapidly insert the catheter tube 110 into the insertion site and advance the catheter tube 110 through the vasculature of the patient without the using the Seldinger technique.

The catheter tube 110 can include a third section 218 proximal of the second section 214 of catheter tube 110 including a bumped diameter demarcated by a bump 217 in the medial portion of the catheter tube 110. The third section 218 of the catheter tube 110 has a larger outer diameter than both the first portion 212 and the second portion 214 of the catheter tube 110. The suture wing 140 can be disposed over the bump 217 as shown among FIGS. 1 and 2. Thus, the catheter tube 110 proximal of the suture wing 140 - also known as a catheter-tube extension herein - has a larger outer diameter than the catheter tube 110 distal of the suture wing 140. The larger outer diameter of the third section 218 of the catheter tube 110 proximal of the suture wing 140 provides a thicker, more kink-resistant catheter-tube wall useful for bending the hub 150 and the number of extension legs 160 away from a head or neck of a patient while the RICC 100 is in use. In addition, any lumens present in the catheter tube 110 can have a greater diameter in the third section 218 of catheter tube 110 proximal of the suture wing 140 than distal of the suture wing 140. This prevents flow rate reduction, particularly when the third section 218 of the catheter tube 110 proximal of the suture wing 140 is bent away from a head or neck of a patient.

The catheter tube 110 between the suture wing 140 and the hub 150 can have a reverse taper in which the larger outer diameter of the catheter tube 110 continues to increase from the suture wing 140 to the hub 150. In other words, the catheter tube 110 tapers from the hub 150 to the suture wing 140 but continues to have a larger outer diameter than the catheter tube 110 distal of the suture wing 140. In association with the continuously increasing outer diameter of the catheter tube 110 from the suture wing 140 to the hub 150, the catheter-tube wall can continuously increase in thickness, any lumens of the catheter tube 110 can continuously increase in cross-sectional area, or a combination thereof. Consequently, the catheter tube 110 between the suture wing 140 and the hub 150 can be more resistant to kinks and flow rate reduction, particularly when the catheter tube 110 proximal of the suture wing 140 is bent away from a head or neck of a patient. Notwithstanding the foregoing, the catheter tube 110 between the suture wing 140 and the hub 150 can alternatively have a constant diameter from the suture wing 140 to the hub 150.

Advantageously, the catheter tube 110 between the suture wing 140 and the hub 150 (e.g., the third section 218 of the catheter tube 110), or the catheter-tube extension, is a single catheter tube configured to abate bacterial ingress between a dressing applied over the suture wing 140 and skin of a patient. Existing CVCs or peripherally inserted central catheters ("PICCs") have multiple extension legs extending from suture wing-hub combinations common to the CVCs and PICCs. The multiple extension legs in the CVCs or PICCs provide multiple pathways under the dressing for microbial ingress. The catheter tube 110 being a single catheter tube between at least the suture wing 140 and the hub 150 enables the dressing to be pinched more tightly around the catheter tube 110 than possible for the multiple extension legs of the existing CVCs or PICCs. For example, the dressing can be easily wrapped around an entirety of the catheter tube 110 and pinched together under the catheter tube 110 between the catheter tube 110 and the patient. In contrast, even wrapping the dressing around the multiple extension legs of the existing CVCs or PICCs as described for the catheter-tube extension leaves gaps between adjacent extension tubes for bacterial ingress. Thus, the catheter tube 110 being a single catheter tube limits bacterial ingress between the dressing applied over the suture wing 140 and the skin of the patient.

The catheter tube 110 between the suture wing 140 and the hub 150, or the catheter-tube extension, is also configured to mitigate patient discomfort from proximity of the number or extension legs 160 to a head or neck of the patient. As set forth above, the third section 218 of the catheter tube 110 proximal of the suture wing 140 provides a thicker, more kink-resistant catheter-tube wall; however, the third section 218 of the catheter tube 110 is flexible enough to enable the catheter tube 110 to be bent away from the head or neck of the patient and secured to the patient for his or her comfort.

FIG. 3 illustrates a distal portion of the catheter tube 110 of the RICC 100 in accordance with some embodiments. FIGS. 4A-4D illustrate various transverse cross sections of the catheter tube in accordance with some embodiments.

Again, the RICC 100 can be a monoluminal catheter or a multiluminal catheter such as a diluminal catheter, a triluminal catheter, a tetraluminal catheter, a pentaluminal catheter, or a hexaluminal catheter. The catheter tube 110 can correspondingly be a monoluminal catheter tube or a multiluminal catheter tube such as a diluminal catheter tube, a triluminal catheter tube, a tetraluminal catheter tube, a pentaluminal catheter tube, or a hexaluminal catheter tube.

When the RICC 100 is configured as a triluminal catheter as shown among FIGS. 1, 2, and 4A-4D, the RICC 100 includes a first lumen, a second lumen, and a third lumen. The first lumen extends from an opening in a proximal end of a first Luer connector of the number of Luer connectors 170 to an opening in a tip or distal end of the first section 212 of the catheter tube 110. The second lumen extends from an opening in a proximal end of a second Luer connector of the number of Luer connectors 170 to an eyelet 320 in a distal portion of the second section 214 of the catheter tube 110. The third lumen extends from an opening in a proximal end of a third Luer connector of the number of Luer connectors 170 to an eyelet 322 proximal of the eyelet 320 in the distal portion of the second section 214 of the catheter tube 110. Each lumen of the first lumen, the second lumen, and the third lumen is further described in a separate paragraph set forth below.

The first lumen of the RICC 100 includes fluidly connected luminal sections including a first catheter-tube lumen 424 extending along an entire length of the catheter tube 110, a first fluid passageway or first cannula lumen of the hub 150, a first extension-leg lumen of a first extension leg (e.g., the extension leg labeled "distal" in FIGS. 1 and 2) of the number of extension legs 160, and a first Luer-connector lumen of the first Luer connector of the number of Luer connectors 170.

The second lumen of the RICC 100 includes fluidly connected luminal sections including a second catheter-tube lumen 426, which proximally extends from the eyelet 320 in the distal portion of the second section 214 of the catheter tube 110 along a remainder of the catheter tube 110. The fluidly connected luminal sections of the second lumen of the RICC 100 further include a second fluid passageway or second cannula lumen of the hub 150, a second extension-leg lumen of a second extension leg (e.g., the extension leg labeled "medial" in FIGS. 1 and 2) of the number of extension legs 160, and a second Luer-connector lumen of the second Luer connector of the number of Luer connectors 170.

The third lumen of the RICC 100 includes fluidly connected luminal sections including a third catheter-tube lumen 428, which proximally extends from the eyelet 322 in the distal portion of the second section 214 of the catheter tube 110 along a remainder of the catheter tube 110. The fluidly connected luminal sections of the third lumen of the RICC 100 further include a third fluid passageway or third cannula lumen of the hub 150, a third extension-leg lumen of a third extension leg (e.g., the extension leg labeled "proximal" in FIGS. 1 and 2) of the number of extension legs 160, and a third Luer-connector lumen of the third Luer connector of the number of Luer connectors 170.

When the RICC 100 is configured as a diluminal catheter, the RICC 100 includes a first lumen and a second lumen. Like the RICC 100 when configured as the triluminal catheter, the first lumen extends from the opening in the proximal end of the first Luer connector of the number of Luer connectors 170 to the opening in the tip or the distal end of the first section 212 of the catheter tube 110. The second lumen extends from the opening in the proximal end of the second Luer connector of the number of Luer connectors 170 to the eyelet 320 in the distal portion of the second section 214 of the catheter tube 110. Because the first lumen and the second lumen of the RICC 100 configured as the diluminal catheter are analogous to the first lumen and the second lumen of the RICC 100 configured as the triluminal catheter, additional detail for each lumen of the first lumen and the second lumen of the RICC 100 configured as the diluminal catheter can be discerned from the description set forth above for the first lumen and the second lumen of the RICC 100 configured as the triluminal catheter.

When the RICC 100 is configured as a monoluminal catheter, the RICC 100 includes a single lumen, which single lumen is also known as a first lumen herein for consistency with description set forth above. Like the RICC 100 when configured as the triluminal catheter, the first lumen extends from the opening in the proximal end of the first Luer connector of the number of Luer connectors 170 to the opening in the tip or the distal end of the first section 212 of the catheter tube 110. Because the first lumen of the RICC 100 configured as the monoluminal catheter is analogous to the first lumen of the RICC 100 configured as the triluminal catheter, additional detail for the first lumen of the RICC 100 configured as the monoluminal catheter can be discerned from the description set forth above for the first lumen of the RICC 100 configured as the triluminal catheter.

### Methods

A method for making the RICC 100 includes one or more steps set forth below for manufacturing the catheter tube 110, one or more extruding steps of extruding one or more extrudable components other than the catheter tube 110 such as the number of extension legs 160, one or more molding steps of molding one or more moldable components, and one or more assembling steps of assembling the RICC 100 or any portion thereof by coupling the extrudable components including the catheter tube 110 and the moldable components together.

The one or more extruding steps can include extruding any one or more extension legs of the number of extension legs 160 in accordance with description set forth above for the one or more extension legs.

The one or more molding steps can include molding any one or more moldable components selected from the suture wing 140 and the hub 150 in accordance with description set forth above for the one or more moldable components. The one or more molding steps can further include molding any one or more Luer connectors of the number of Luer connectors 170 in accordance with description set forth above for the one or more Luer connectors.

The one or more assembling steps of assembling the RICC 100 or any portion thereof can include assembling the RICC 100 in accordance with that shown in FIG. 2 with the understanding a distal-end portion of the catheter tube 110 is inserted into a proximal-end portion the suture wing 140.

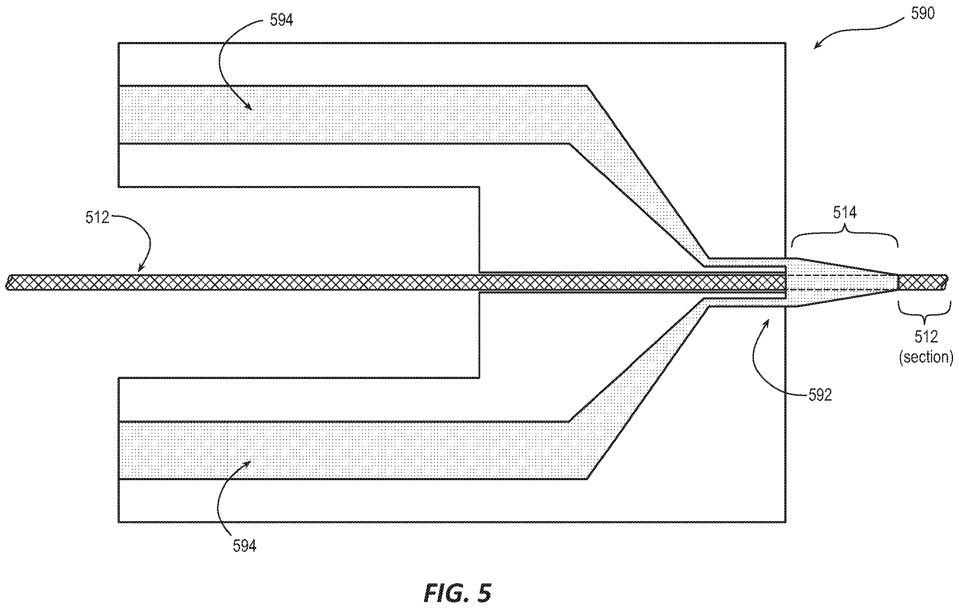

FIG. 5 illustrates a portion of a method of manufacturing the catheter tube 110 in accordance with some embodiments.

A method for manufacturing layered catheter tubes such as the catheter tube 110 includes an inner-layer forming step of forming the inner layer 412 of the catheter tube 110 by extruding monoluminal tubing 512 of the first polymeric material.

The method further includes an inserting step of inserting an end of the monoluminal tubing 512 through a die 592 of an extruder 590.

The method further includes a second-layer forming step of forming the outer layer 414 of the catheter tube 110 by periodically forcing a melt 594 of the second polymeric material through the die 592 around the monoluminal tubing 512, thereby forming mixed-layer tubing having sections of the monoluminal tubing 512 regularly interspersed with sections of layered tubing 514.

The method can further include a bonding layer-applying step of applying a bonding layer over the monoluminal tubing 512 before forcing the melt 594 of the second polymeric material through the die 592 around the monoluminal tubing 512 in the second-layer forming step.

The method further includes a lumen forming step of forming one or more additional lumens (e.g., the second catheter-tube lumen 426, the third catheter-tube lumen 428, etc.) to that of the monoluminal tubing 512 by injecting air into the melt 594 of the second polymeric material while forcing the melt 594 of the second polymeric material through the die 592 around the monoluminal tubing 512.

The method further includes an eyelet-creating step of creating one or more eyelets (e.g., the eyelet 320, the eyelet 322, etc.) in the sections of the layered tubing 514 to correspondingly establish one or openings to the one or more additional lumens.

The method can further include a bump-forming step of forming bumps (e.g., the bump 217 in the medial portion of the catheter tube 110) in outer diameter in the sections of the layered tubing 514 by periodically slowing a rate of pulling the mixed-layer tubing with the puller to increase the outer diameter after the bumps.

The method can further include a reverse-tapering step of reverse tapering the outer diameter in the sections of the layered tubing 514 after the bumps by continuously slowing the rate of pulling the mixed-layer tubing with the puller.

The method can further include a pulling step of pulling the mixed-layer tubing through a cooling bath with a puller to cool the mixed-layer tubing.

The method further includes a cutting step of cutting the mixed-layer tubing in at least the sections of the monoluminal tubing 512 with a cutter to form the layered catheter tubes.

A method of using the RICC 100 includes a creating step of creating an insertion site to access a vasculature of a patient with a needle disposed within a lumen of the RICC 100. The insertion site can be at a subclavian vein such as a right or left subclavian vein, an internal jugular vein such as a right or left internal jugular vein, or a femoral vein.

The method further includes an inserting step of inserting the distal-end portion of the catheter tube 110 into the insertion site.

The method further includes a withdrawing step of withdrawing the needle from the lumen of the RICC 100 after creating the insertion site and inserting at least some of the first section 212 of the catheter tube 110 into the insertion site.

The method further includes an advancing step of advancing the catheter tube 110 through the vasculature of the patient without having to use the Seldinger technique. For example, if the insertion site is at the right subclavian vein or the right internal jugular vein, the advancing step can further include inserting the catheter tube 110 farther into the insertion site such that the catheter tube 110 or at least the distal-end portion thereof is advanced through the right subclavian vein or the right internal jugular vein, a right brachiocephalic vein, and into a superior vena cava. Other insertions sites such as at the left subclavian vein or the left internal jugular vein require advancing the distal-end portion of the catheter tube 110 through corresponding vasculature. The Seldinger technique need not be used due to the catheter tube 110 having a column strength sufficient to prevent buckling of the catheter tube 110 when inserted into the insertion site and advanced through the vasculature of the patient.

## Claims

1. A catheter tube (110), comprising:
a first section (212) in a distal portion of the catheter tube (110); and
a second section (214) proximal of the first section (212) including an outer layer (414) of the catheter tube (110) extruded over an inner layer (412) of the catheter tube (110) such that an outer diameter of the catheter tube (110) is larger in the second section (214) than the first section (212), **characterized in that** the inner layer (412) of the catheter tube (110) is formed of a first polymeric material having a first durometer and the outer layer (414) of the catheter tube (110) is formed of a second polymeric material having a second durometer less than or substantially equal to the first durometer.

2. The catheter tube (110) of claim 1, wherein the catheter tube (110) further includes a bump (217) demarcating a third section (218) of the catheter tube (110) proximal of the second section (214), the third section (218) having a larger outer diameter than both the first and second sections (212, 214) of the catheter tube (110), and wherein the catheter tube (110) between the bump (217) and a proximal end of the third section (218) of catheter tube (110) has a reverse taper in which the outer diameter of the catheter tube (110) continuously increases from the bump (217) to the proximal end of the third section (218) of catheter tube (110).

3. The catheter tube (110) of either claim 1 or 2, wherein the catheter tube (110) is a triluminal catheter tube including a first lumen (424) extending from a first opening in a proximal end of the catheter tube (110) to an opening in a distal end of the first section (212) of the catheter tube (110), a second lumen (426) extending from a second opening in the proximal end of the catheter tube (110) to a first eyelet in a distal portion of the second section (214) of the catheter tube (110), and a third lumen (428) extending from a third opening in the proximal end of the catheter tube (110) to a second eyelet in the distal portion of the second section (214) of the catheter tube (110), or
wherein the catheter tube is a diluminal catheter tube including a first lumen (424) extending from a first opening in a proximal end of the catheter tube (110) to an opening in a distal end of the first section (212) of the catheter tube (110) and a second lumen (426) extending from a second opening in the proximal end of the catheter tube (110) to an eyelet in a distal portion of the second section (214) of the catheter tube (110).

4. A rapidly insertable central catheter ("RICC") (100), comprising:
a catheter tube (110) according to any one of claims 1-3;
a suture wing (140) disposed over a medial portion of the catheter tube (110);
a hub (150) coupled to a proximal-end portion of the catheter tube (110); and
a number of extension legs (160) extending from the hub equal to a number of lumens (424, 426, 428) extending through the RICC (100).

5. The RICC (100) of claim 4, wherein each polymeric material of the first and second polymeric materials is a polyurethane.

6. The RICC (100) of claim 4, wherein each layer of the inner and outer layers (412, 414) of the catheter tube (110) is formed of a same polymeric material, and wherein, preferably, the polymeric material is a polyurethane.

7. The RICC (100) of any claim of claims 4-6, wherein the catheter tube (110) further includes a bump (217) demarcating a third section (218) of the catheter tube (110) proximal of the second section (214), the third section (218) having a larger outer diameter than both the first and second sections (212, 214) of the catheter tube (110), and wherein, preferably, the suture wing (140) is disposed over the bump (217) of the catheter tube (110) such that the catheter tube (110) proximal of the suture wing (140) has a larger outer diameter than the catheter tube (110) distal of the suture wing (140).

8. The RICC (100) of any claim of claims 4-6, wherein the catheter tube (110) between the suture wing (140) and the hub (150) has a reverse taper in which the outer diameter of the catheter tube (110) continuously increases from the suture wing (140) to the hub (150), and/or
wherein the catheter tube (110) has a column strength sufficient to prevent buckling of the catheter tube (110) when inserted into an insertion site and advanced through a vasculature of a patient.

9. The RICC (100) of any claim of claims 4-8, wherein the RICC (100) is a triluminal catheter including a trifurcated hub as the hub having three extension legs (160) extending therefrom, each extension leg (160) of the three extension legs (160) including a Luer connector (170) coupled to a proximal-end portion of the extension leg (160), and wherein, preferably, the RICC (100) includes a first lumen (424) extending from an opening in a proximal end of a first Luer connector (170) to an opening in a distal end of the first section (212) of the catheter tube (110), a second lumen (426) extending from an opening in a proximal end of a second Luer connector (170) to a first eyelet in a distal portion of the second section (214) of the catheter tube (110), and a third lumen (428) extending from an opening in a proximal end of a third Luer connector (170) to a second eyelet in the distal portion of the second section (214) of the catheter tube (110).

10. The RICC (100) of any claim of claims 4-9, wherein the RICC (100) is a diluminal catheter including a bifurcated hub as the hub having two extension legs (160) extending therefrom, each extension leg (160) of the two extension legs (160) including a Luer connector (170) coupled to a proximal-end portion of the extension leg (160), and wherein, preferably, the RICC (100) includes a first lumen (424) extending from an opening in a proximal end of a first Luer connector (170) to an opening in a distal end of the first section (212) of the catheter tube (110) and a second lumen (426) extending from an opening in a proximal end of a second Luer connector (170) to an eyelet in a distal portion of the second section (214) of the catheter tube (110).

11. A method for manufacturing the layered catheter tube (110) of any one of claims 1-3, comprising:
forming an inner layer (412) of the catheter tube (110) by extruding monoluminal tubing (512) of a first polymeric material;
inserting an end of the monoluminal tubing (512) through a die (592) of an extruder (590);
forming an outer layer (414) of the catheter tube (110) by periodically forcing a melt of a second polymeric material through the die (592) around the monoluminal tubing (512), thereby forming mixed-layer tubing having sections of the monoluminal tubing (512) interspersed with sections of layered tubing (514);
pulling the mixed-layer tubing through a cooling bath with a puller to cool the mixed-layer tubing; and
cutting the mixed-layer tubing in at least the sections of the monoluminal tubing (512) with a cutter to form the layered catheter tubes (110).

12. The method of claim 11, further comprising forming one or more additional lumens to that of the monoluminal tubing (512) by injecting air into the melt of the second polymeric material while forcing the melt of the second polymeric material through the die (592) around the monoluminal tubing (512),
the method preferably further comprising creating one or more eyelets in the sections of the layered tubing (514) to correspondingly establish one or openings to the one or more additional lumens.

13. The method of either claim 11 or 12, further comprising forming bumps (217) in outer diameter in the sections of the layered tubing (514) by periodically slowing a rate of pulling the mixed-layer tubing with the puller to increase the outer diameter after the bumps (217),
the method preferably further comprising reverse tapering the outer diameter in the sections of the layered tubing (514) after the bumps by continuously slowing the rate of pulling the mixed-layer tubing with the puller.

14. The method of any claim of claims 11-13, further comprising applying a bonding layer over the monoluminal tubing (512) before forcing the melt of the second polymeric material through the die (592) around the monoluminal tubing (512).

15. The method of any claim of claims 11-14, wherein the first polymeric material has a first durometer and the second polymeric material has a second durometer less than the first durometer, or
wherein the first polymeric material has a first durometer and the second polymeric material has a second durometer substantially equal to the first durometer, and
wherein, preferably, each polymeric material of the first and second polymeric materials is a polyurethane.

## Patentansprüche

1. Katheterschlauch (110), umfassend:
ein erstes Teilstück (212) in einem distalen Abschnitt des Katheterschlauchs (110); und
ein zweites Teilstück (214) proximal von dem ersten Teilstück (212), das eine über eine Innenschicht (412) des Katheterschlauchs (110) extrudierte Außenschicht (414) des Katheterschlauchs (110) einschließt, sodass ein Außendurchmesser des Katheterschlauchs (110) in dem zweiten Teilstück (214) größer als in dem ersten Teilstück (212) ist, **dadurch gekennzeichnet, dass** die Innenschicht (412) des Katheterschlauchs (110) aus einem ersten polymeren Material, das eine erste Durometerhärte aufweist, gebildet ist und die Außenschicht (414) des Katheterschlauchs (110) aus einem zweiten polymeren Material, das eine zweite Durometerhärte kleiner als oder im Wesentlichen gleich der ersten Durometerhärte aufweist, gebildet ist.

2. Katheterschlauch (110) nach Anspruch 1, wobei der Katheterschlauch (110) weiter eine Verdickung (217) einschließt, die ein drittes Teilstück (218) des Katheterschlauchs (110) proximal von dem zweiten Teilstück (214) abgrenzt, wobei das dritte Teilstück (218) einen größeren Außendurchmesser als sowohl das erste als auch das zweite Teilstück (212, 214) des Katheterschlauchs (110) aufweist, und wobei der Katheterschlauch (110) zwischen der Verdickung (217) und einem proximalen Ende des dritten Teilstücks (218) des Katheterschlauchs (110) eine umgekehrte Verjüngung aufweist, bei der der Außendurchmesser des Katheterschlauchs (110) von der Verdickung (217) zu dem proximalen Ende des dritten Teilstücks (218) des Katheterschlauchs (110) kontinuierlich zunimmt.

3. Katheterschlauch (110) nach Anspruch 1 oder 2, wobei der Katheterschlauch (110) ein dreilumiger Katheterschlauch ist, der ein erstes Lumen (424), das sich von einer ersten Öffnung in einem proximalen Ende des Katheterschlauchs (110) zu einer Öffnung in einem distalen Ende des ersten Teilstücks (212) des Katheterschlauchs (110) erstreckt, ein zweites Lumen (426), das sich von einer zweiten Öffnung in dem proximalen Ende des Katheterschlauchs (110) zu einem ersten Katheterauge in einem distalen Abschnitt des zweiten Teilstücks (214) des Katheterschlauchs (110) erstreckt, und ein drittes Lumen (428), das sich von einer dritten Öffnung in dem proximalen Ende des Katheterschlauchs (110) zu einem zweiten Katheterauge in dem distalen Abschnitt des zweiten Teilstücks (214) des Katheterschlauchs (110) erstreckt, einschließt, oder wobei der Katheterschlauch ein doppellumiger Katheterschlauch ist, der ein erstes Lumen (424), das sich von einer ersten Öffnung in einem proximalen Ende des Katheterschlauchs (110) zu einer Öffnung in einem distalen Ende des ersten Teilstücks (212) des Katheterschlauchs (110) erstreckt, und ein zweites Lumen (426), das sich von einer zweiten Öffnung in dem proximalen Ende des Katheterschlauchs (110) zu einem Katheterauge in einem distalen Abschnitt des zweiten Teilstücks (214) des Katheterschlauchs (110) erstreckt, einschließt.

4. Schnell einführbarer zentraler Katheter ("RICC") (100), umfassend:
einen Katheterschlauch (110) nach einem der Ansprüche 1-3;
einen Nahtflügel (140), der über einem mittleren Abschnitt des Katheterschlauchs (110) angeordnet ist;
eine Nabe (150), die an einen proximalen Endabschnitt des Katheterschlauchs (110) gekoppelt ist; und
eine Anzahl von Verlängerungsschenkeln (160), die sich von der Nabe erstrecken, die gleich einer Anzahl von Lumen (424, 426, 428) ist, die sich durch den RICC (100) erstrecken.

5. RICC (100) nach Anspruch 4, wobei jedes polymere Material des ersten und des zweiten polymeren Materials ein Polyurethan ist.

6. RICC (100) nach Anspruch 4, wobei jede Schicht der Innen- und der Außenschicht (412, 414) des Katheterschlauchs (110) aus demselben polymeren Material gebildet ist und wobei das polymere Material vorzugsweise ein Polyurethan ist.

7. RICC (100) nach einem der Ansprüche 4-6, wobei der Katheterschlauch (110) weiter eine Verdickung (217) einschließt, die ein drittes Teilstück (218) des Katheterschlauchs (110) proximal von dem zweiten Teilstück (214) abgrenzt, wobei das dritte Teilstück (218) einen größeren Außendurchmesser als sowohl das erste als auch das zweite Teilstück (212, 214) des Katheterschlauchs (110) aufweist, und wobei der Nahtflügel (140) vorzugsweise über der Verdickung (217) des Katheterschlauchs (110) angeordnet ist, sodass der Katheterschlauch (110) proximal von dem Nahtflügel (140) einen größeren Außendurchmesser als der Katheterschlauch (110) distal von dem Nahtflügel (140) aufweist.

8. RICC (100) nach einem der Ansprüche 4-6, wobei der Katheterschlauch (110) zwischen dem Nahtflügel (140) und der Nabe (150) eine umgekehrte Verjüngung aufweist, bei der der Außendurchmesser des Katheterschlauchs (110) von dem Nahtflügel (140) zu der Nabe (150) kontinuierlich zunimmt, und/oder
wobei der Katheterschlauch (110) eine Säulenfestigkeit aufweist, die ausreicht, um ein Knicken des Katheterschlauchs (110) zu verhindern, wenn er in eine Einsetzstelle eingesetzt und durch ein Gefäßsystem eines Patienten vorgeschoben wird.

9. RICC (100) nach einem der Ansprüche 4-8, wobei der RICC (100) ein dreilumiger Katheter ist, der eine trifurkierte Nabe als die Nabe einschließt, die drei Verlängerungsschenkel (160) aufweist, die sich davon erstrecken, wobei jeder Verlängerungsschenkel (160) der drei Verlängerungsschenkel (160) einen Luer-Verbinder (170) aufweist, der an einen proximalen Endabschnitt des Verlängerungsschenkels (160) gekoppelt ist, und wobei der RICC (100) vorzugsweise ein erstes Lumen (424), das sich von einer Öffnung in einem proximalen Ende eines ersten Luer-Verbinders (170) zu einer Öffnung in einem distalen Ende des ersten Teilstücks (212) des Katheterschlauchs (110) erstreckt, ein zweites Lumen (426), das sich von einer Öffnung in einem proximalen Ende eines zweiten Luer-Verbinders (170) zu einem ersten Katheterauge in einem distalen Abschnitt des zweiten Teilstücks (214) des Katheterschlauchs (110) erstreckt, und ein drittes Lumen (428), das sich von einer Öffnung in einem proximalen Ende eines dritten Luer-Verbinders (170) zu einem zweiten Katheterauge in dem distalen Abschnitt des zweiten Teilstücks (214) des Katheterschlauchs (110) erstreckt, einschließt.

10. RICC (100) nach einem der Ansprüche 4-9, wobei der RICC (100) ein doppellumiger Katheter ist, der eine bifurkierte Nabe als die Nabe einschließt, die zwei Verlängerungsschenkel (160) aufweist, die sich davon erstrecken, wobei jeder Verlängerungsschenkel (160) der zwei Verlängerungsschenkel (160) einen Luer-Verbinder (170) einschließt, der an einen proximalen Endabschnitt des Verlängerungsschenkels (160) gekoppelt ist, und wobei der RICC (100) vorzugsweise ein erstes Lumen (424), das sich von einer Öffnung in einem proximalen Ende eines ersten Luer-Verbinders (170) zu einer Öffnung in einem distalen Ende des ersten Teilstücks (212) des Katheterschlauchs (110) erstreckt, und ein zweites Lumen (426), das sich von einer Öffnung in einem proximalen Ende eines zweiten Luer-Verbinders (170) zu einem Katheterauge in einem distalen Abschnitt des zweiten Teilstücks (214) des Katheterschlauchs (110) erstreckt, einschließt.

11. Verfahren zum Herstellen des geschichteten Katheterschlauchs (110) nach einem der Ansprüche 1-3, umfassend:
Bilden einer Innenschicht (412) des Katheterschlauchs (110) durch Extrudieren eines einlumigen Schlauchstücks (512) aus einem ersten polymeren Material;
Einführen eines Endes des einlumigen Schlauchstücks (512) durch eine Extrusionsdüse (592) eines Extruders (590);
Bilden einer Außenschicht (414) des Katheterschlauchs (110) durch periodisches Pressen einer Schmelze eines zweiten polymeren Materials durch die Extrusionsdüse (592) um das einlumige Schlauchstück (512) herum, wodurch ein Schlauchstück mit gemischten Schichten gebildet wird, das Teilstücke des einlumigen Schlauchstücks (512) aufweist, die mit Teilstücken von geschichteten Schlauchstücken (514) durchsetzt sind;
Ziehen des Schlauchstücks mit gemischten Schichten durch ein Kühlbad mit einer Zieheinrichtung, um das Schlauchstück mit gemischten Schichten abzukühlen; und
Schneiden des Schlauchstücks mit gemischten Schichten mindestens in den Teilstücken des einlumigen Schlauchstücks (512) mit einem Schneidwerkzeug, um die geschichteten Katheterschläuche (110) zu bilden.

12. Verfahren nach Anspruch 11, weiter umfassend das Bilden eines oder mehrerer zusätzlicher Lumen zu dem des einlumigen Schlauchstücks (512) durch Einspritzen von Luft in die Schmelze des zweiten polymeren Materials, während die Schmelze des zweiten polymeren Materials durch die Extrusionsdüse (592) um das einlumige Schlauchstück (512) herum gepresst wird,
wobei das Verfahren vorzugsweise weiter das Erschaffen eines oder mehrerer Katheteraugen in den Teilstücken des geschichteten Schlauchstücks (514), um entsprechend eine oder mehrere Öffnungen zu dem einen oder den mehreren zusätzlichen Lumen herzustellen, umfasst.

13. Verfahren nach Anspruch 11 oder 12, weiter umfassend das Bilden von Verdickungen (217) in dem Außendurchmesser in den Teilstücken des geschichteten Schlauchstücks (514) durch periodisches Verlangsamen einer Zugrate des Schlauchstücks mit gemischten Schichten mit der Zugeinrichtung, um den Außendurchmesser nach den Verdickungen (217) zu vergrößern,
wobei das Verfahren vorzugsweise weiter das Bilden einer umgekehrten Verjüngung des Außendurchmessers in den Teilstücken des geschichteten Schlauchstücks (514) nach den Verdickungen durch kontinuierliches Verlangsamen der Zugrate des Schlauchstücks mit gemischten Schichten mit der Zugeinrichtung umfasst.

14. Verfahren nach einem der Ansprüche 11-13, weiter umfassend das Aufbringen einer Bindungsschicht über das einlumige Schlauchstück (512) vor dem Pressen der Schmelze des zweiten polymeren Materials durch die Extrusionsdüse (592) um das einlumige Schlauchstück (512) herum.

15. Verfahren nach einem der Ansprüche 11-14, wobei das erste polymere Material eine erste Durometerhärte aufweist und das zweite polymere Material eine zweite Durometerhärte kleiner als die erste Durometerhärte aufweist, oder
wobei das erste polymere Material eine erste Durometerhärte aufweist und das zweite polymere Material eine zweite Durometerhärte im Wesentlichen gleich der ersten Durometerhärte aufweist, und
wobei jedes polymere Material des ersten und des zweiten polymeren Materials vorzugsweise ein Polyurethan ist.

## Revendications

1. Tube (110) de cathéter, comprenant :
une première section (212) dans une partie distale du tube (110) de cathéter ; et
une deuxième section (214) proximale à la première section (212) incluant une couche externe (414) du tube (110) de cathéter extrudée sur une couche interne (412) du tube (110) de cathéter de telle sorte qu'un diamètre externe du tube (110) de cathéter est plus grand dans la deuxième section (214) que dans la première section (212), **caractérisé en ce que** la couche interne (412) du tube (110) de cathéter est formée d'un premier matériau polymère présentant un premier duromètre et la couche externe (414) du tube (110) de cathéter est formée d'un deuxième matériau polymère présentant un deuxième duromètre inférieur ou sensiblement égal au premier duromètre.

2. Tube (110) de cathéter selon la revendication 1, dans lequel le tube (110) de cathéter inclut en outre un renflement (217) délimitant une troisième section (218) du tube (110) de cathéter proximale à la deuxième section (214), la troisième section (218) présentant un diamètre externe plus grand que celui des première et deuxième (212, 214) du tube (110) de cathéter, et dans lequel le tube (110) de cathéter entre le renflement (217) et une extrémité proximale de la troisième section (218) du tube (110) de cathéter présente un rétrécissement inverse dans lequel le diamètre externe du tube (110) de cathéter augmente en continu depuis le renflement (217) jusqu'à l'extrémité proximale de la troisième section (218) du tube (110) de cathéter.

3. Tube (110) de cathéter selon la revendication 1 ou la revendication 2, dans lequel le tube (110) de cathéter est un tube de cathéter trilumière incluant une première lumière (424) s'étendant depuis une première ouverture dans une extrémité proximale du tube (110) de cathéter jusqu'à une ouverture dans une extrémité distale de la première section (212) du tube (110) de cathéter, une deuxième lumière (426) s'étendant depuis une deuxième ouverture dans l'extrémité proximale du tube (110) de cathéter jusqu'à un premier œillet dans une partie distale de la deuxième section (214) du tube (110) de cathéter, et une troisième lumière (428) s'étendant depuis une troisième ouverture dans l'extrémité proximale du tube (110) de cathéter jusqu'à un deuxième œillet dans la partie distale de la deuxième section (214) du tube (110) de cathéter, ou
dans lequel le tube de cathéter est un tube de cathéter bilumière comprenant une première lumière (424) s'étendant depuis une première ouverture dans une extrémité proximale du tube (110) de cathéter jusqu'à une ouverture dans une extrémité distale de la première section (212) du tube (110) de cathéter et une deuxième lumière (426) s'étendant depuis une deuxième ouverture dans l'extrémité proximale du tube (110) de cathéter jusqu'à un œillet dans une partie distale de la deuxième section (214) du tube (110) de cathéter.

4. Cathéter central à insertion rapide (« RICC ») (100), comprenant :
un tube (110) de cathéter selon l'une quelconque des revendications 1 à 3 ;
une ailette de suture (140) disposée sur une partie médiane du tube (110) de cathéter;
un raccord (150) couplé à une partie d'extrémité proximale du tube (110) de cathéter ; et
un nombre de pattes d'extension (160) s'étendant depuis le raccord égal à un nombre de lumières (424, 426, 428) s'étendant à travers le RICC (100).

5. RICC (100) selon la revendication 4, dans lequel chaque matériau polymère des premier et deuxième matériaux polymères est un polyuréthane.

6. RICC (100) selon la revendication 4, dans lequel chaque couche des couches interne et externe (412, 414) du tube (110) de cathéter est formée d'un même matériau polymère, et dans lequel, de préférence, le matériau polymère est un polyuréthane.

7. RICC (100) selon l'une quelconque des revendications 4 à 6, dans lequel le tube (110) de cathéter inclut en outre un renflement (217) délimitant une troisième section (218) du tube (110) de cathéter proximale à la deuxième section (214), la troisième section (218) présentant un diamètre externe plus grand que celui des première et deuxième (212, 214) du tube (110) de cathéter, et dans lequel, de préférence, l'ailette de suture (140) est disposée sur le renflement (217) du tube (110) de cathéter de telle sorte que le tube (110) de cathéter est proximal à l'ailette de suture (140) présente un diamètre externe plus grand que le tube (110) de cathéter distal à l'ailette de suture (140).

8. RICC (100) selon l'une quelconque des revendications 4 à 6, dans lequel le tube (110) de cathéter entre l'ailette de suture (140) et le raccord (150) présente un rétrécissement inverse dans lequel le diamètre externe du tube (110) de cathéter augmente continuellement depuis l'ailette de suture (140) jusqu'au raccord (150), et/ou
dans lequel le tube (110) de cathéter présente une résistance en colonne suffisante pour empêcher la déformation du tube (110) de cathéter lorsqu'il est inséré dans un site d'insertion et avancé à travers un système vasculaire d'un patient.

9. RICC (100) selon l'une quelconque des revendications 4 à 8, dans lequel le RICC (100) est un cathéter trilumière incluant un raccord trifurqué en tant que raccord présentant trois pattes d'extension (160) s'étendant à partir de celui-ci, chaque patte d'extension (160) des trois pattes d'extension (160) incluant un embout Luer (170) couplé à une partie d'extrémité proximale de la patte d'extension (160), et dans lequel, de préférence, le RICC (100) inclut une première lumière (424) s'étendant depuis une ouverture dans une extrémité proximale d'un premier embout Luer (170) jusqu'à une ouverture dans une extrémité distale de la première section (212) du tube (110) de cathéter, une deuxième lumière (426) s'étendant depuis une ouverture dans une extrémité proximale d'un deuxième embout Luer (170) jusqu'à un premier œillet dans une partie distale de la deuxième section (214) du tube (110) de cathéter, et une troisième lumière (428) s'étendant depuis une ouverture dans une extrémité proximale d'un troisième embout Luer (170) jusqu'à un deuxième œillet dans la partie distale de la deuxième section (214) du tube (110) de cathéter.

10. RICC (100) selon l'une quelconque des revendications 4 à 9, dans lequel le RICC (100) est un cathéter bilumière comprenant un raccord bifurqué en tant que raccord présentant deux pattes d'extension (160) s'étendant à partir de celui-ci, chaque patte d'extension (160) des deux pattes d'extension (160) incluant un embout Luer (170) couplé à une partie d'extrémité proximale de la patte d'extension (160), et dans lequel, de préférence, le RICC (100) inclut une première lumière (424) s'étendant depuis une ouverture dans une extrémité proximale d'un premier embout Luer (170) jusqu'à une ouverture dans une extrémité distale de la première section (212) du tube (110) de cathéter et une deuxième lumière (426) s'étendant depuis une ouverture dans une extrémité proximale d'un deuxième embout Luer (170) jusqu'à un œillet dans une partie distale de la deuxième section (214) du tube (110) de cathéter.

11. Procédé de fabrication du tube (110) de cathéter stratifié selon l'une quelconque des revendications 1 à 3, comprenant :
la formation d'une couche interne (412) du tube (110) de cathéter par extrusion d'un tubage monolumière (512) d'un premier matériau polymère ;
l'insertion d'une extrémité du tubage monolumière (512) à travers une filière (592) d'une extrudeuse (590) ;
la formation d'une couche externe (414) du tube (110) de cathéter par forçage périodique d'une matière fondue d'un deuxième matériau polymère à travers la filière (592) autour du tubage monolumière (512), formant ainsi un tubage à couches mixtes présentant des sections du tubage monolumière (512) intercalées avec des sections de tubage stratifié (514) ;
la traction du tubage à couches mixtes à travers un bain de refroidissement à l'aide d'un dispositif de traction pour refroidir le tubage à couches mixtes ; et
la découpe du tubage à couches mixtes en au moins les sections du tubage monolumière (512) à l'aide d'un outil de coupe pour former les tubes (110) de cathéter stratifiés.

12. Procédé selon la revendication 11, comprenant en outre la formation d'une ou plusieurs lumières supplémentaires par rapport à celle du tubage monolumière (512) par injection d'air dans la matière fondue du deuxième matériau polymère tout en forçant la matière fondue du deuxième matériau polymère à travers la filière (592) autour du tubage monolumière (512), le procédé comprenant de préférence en outre la création d'un ou de plusieurs œillets dans les sections du tubage stratifié (514) pour établir de manière correspondante une ou plusieurs ouvertures vers les une ou plusieurs lumières supplémentaires.

13. Procédé selon la revendication 11 ou la revendication 12, comprenant en outre la formation de renflements (217) en diamètre externe dans les sections du tubage stratifié (514) par ralentissement périodique d'une vitesse de traction du tubage à couches mixtes à l'aide du dispositif de traction pour augmenter le diamètre externe après les renflements (217), le procédé comprenant en outre de préférence le rétrécissement inverse du diamètre externe dans les sections du tubage stratifié (514) après les renflements par ralentissement continu de la vitesse de traction du tubage à couches mixtes à l'aide du dispositif de traction.

14. Procédé selon l'une quelconque des revendications 11 à 13, comprenant en outre l'application d'une couche de liaison sur le tubage monolumière (512) avant de forcer la matière fondue du deuxième matériau polymère à travers la filière (592) autour du tubage monolumière (512).

15. Procédé selon l'une quelconque des revendications 11 à 14, dans lequel le premier matériau polymère présente un premier duromètre et le deuxième matériau polymère présente un deuxième duromètre inférieur au premier duromètre, ou
dans lequel le premier matériau polymère présente un premier duromètre et le deuxième matériau polymère présente un deuxième duromètre sensiblement égal au premier duromètre, et
dans lequel, de préférence, chaque matériau polymère des premier et deuxième matériaux polymères est un polyuréthane.
